# EUROPEAN PATENT APPLICATION

(11) **EP 4 349 320 A1**
(43) Date of publication of application: **10.04.2024**
(21) Application number: 22816312.7
(22) Date of filing: 02.05.2022
(51) Int. Cl.: A61K 8/06, A61K 8/19, A61K 8/24, A61K 8/73, A61Q 19/00

(54) **MULTILAYER EMULSION COSMETIC COMPOSITION**

(30) Priority: 03.06.2021 KR 20210072089
(71) Applicant: Kolmar Korea Co., Ltd., Sejong 30004 (KR)
(72) Inventor: JEONG, Eun Sook, Seoul 06800 (KR); BAE, Hyung Jin, Seoul 06800 (KR); KIM, Jin Mo, Seoul 06800 (KR); HONG, In Ki, Seoul 06800 (KR)
(74) Representative: Nordic Patent Service A/S
(86) International application number: PCT/KR2022/006234
(87) International publication number: WO 2022/255653

(57) **Abstract**

The present invention relates to a multilayer emulsion cosmetic composition which does not contain a surfactant and which exhibits excellent stability.

## Description

### TECHNICAL FIELD

The present invention relates to a cosmetic composition, and specifically, to a multilayer emulsion cosmetic composition which includes an inner phase of a hydrogel layer, an intermediate phase of an organogel layer, and an outer phase of a water layer.

### BACKGROUND ART

Various types of emulsion-type cosmetics are used to provide various active ingredients to the skin, and emulsion-type cosmetics are generally classified into an oil-in-water (O/W) type formulation and a water-in-oil (W/O) type formulation. Since these emulsion-type cosmetics include an oil phase and a water phase, they can provide a softening effect and a moisturizing effect. However, since the external surface of the oil-in-water type formulation is water, it provides a refreshing feeling when applied and has an excellent application property, but has limitations in that it has poor makeup durability and water resistance. In contrast, the water-in-oil type formulation has excellent makeup durability and water resistance, but has limitations in that it does not provide a refreshing feeling due to stickiness, oily feeling, *etc.* and has a poor feeling of use, such as application and spreadability. In addition, in the case of unstable ingredients among various active ingredients, it is difficult to stably support them in conventional emulsified cosmetics, therefore, there are problems such as precipitation or separation of active ingredients in the formulation.

As an alternative to these conventional emulsion-type cosmetics, studies have been conducted on water-in-oil-in-water (W/O/W) type multilayer emulsion cosmetics having the advantages of both O/W and W/O type cosmetics. However, the W/O/W type cosmetics are those in which an oil phase exists between the inner and outer water phases, and the W/O/W type cosmetics have a limitation in that the interfacial film can easily be destroyed or the phase conversion phenomenon may occur due to the osmotic pressure phenomenon caused by a difference in electrolytic concentration between the outer and inner water phases, thus having a difficulty in securing stability. Surfactants are commonly used to properly adjust the ratio of the water phase and the oil phase to realize excellent feeling of use and stability of the interfacial film, but the use of a surfactant has a limitation in that it is against the recent consumer preference for ingredients which should be safe for the skin and the environment. Therefore, there is a demand for the development of a multilayer emulsion cosmetic composition, which has excellent stability of the interfacial film between the water phase and the oil phase without containing a surfactant.

### SUMMARY OF THE INVENTION

The present invention provides a multilayer emulsion cosmetic composition which does not contain a surfactant and exhibits excellent stability.

The multilayer emulsion cosmetic composition of the present invention includes an inner phase of a hydrogel layer, an intermediate phase of an organogel layer, and an outer phase of a water layer.

The present invention provides a multilayer emulsion cosmetic composition that exhibits excellent stability due to high degree of stability of an interfacial film between a water phase and an oil phase even without containing a surfactant. Since the cosmetic composition according to the present invention does not contain a surfactant, it is less irritating to the skin and is environmentally-friendly, and as a multilayer emulsion formulation, has excellent properties of makeup durability and water resistance as well as application, spreadability, and feeling of use. In addition, the cosmetic composition of the present invention is suitable for simultaneously stabilizing the active ingredients consisting of oil-soluble and water-soluble portions into one formulation because the intermediate phase and the inner phase are not brought into contact with air due to the structural characteristics of the multilayer emulsion.

### BRIEF DESCRIPTION OF THE DRAWINGS

FIG. 1 shows a microscopic image of an intermediate phase according to Example 1.
FIG. 2 shows a microscopic image of an intermediate phase of according to Comparative Example 1.
FIG. 3 shows a microscopic image of an intermediate phase of according to Comparative Example 3.
FIG. 4 shows a microscopic image of a W/O type formulation according to Example 4.
FIG. 5 shows a microscopic image of a W/O type formulation according to Example 5.
FIG. 6 shows a microscopic image of a W/O type formulation according to Example 6.
FIG. 7 shows a microscopic image of a W/O/W type formulation according to Example 7.

### DETAILED DESCRIPTION

Hereinafter, the present invention will be described. However, the present invention is not limited merely by the following descriptions, and each component may variously be modified or selectively mixed as necessary. Therefore, it should be understood that the present invention includes all modifications, equivalents, or substitutes included in the spirit and scope of the present invention.

The present invention relates to a multilayer emulsion cosmetic composition, which includes an inner phase of a hydrogel layer, an intermediate phase of an organogel layer, and an outer phase of a water layer.

### Inner Phase

The inner layer of the multilayer emulsion cosmetic composition according to the present invention is a hydrogel layer and it includes purified water, a hydrogel-forming material, and a salt.

As the hydrogel-forming material, any material known to form a hydrogel structure in the art may be used. For example, the hydrogel-forming material includes gellan gum, agar, carrageenan, alginate, pectin, gelatin, xanthan gum, locust bean gum (LBG), guar gum, starch, chitosan, cellulose, collagen, *etc.,* and these may be used alone or in combination of two or more.

The hydrogel-forming material may be contained in an amount of 0.05 wt% to 10 wt%, for example, 0.1 wt% to 5% wt% based on the total weight of the inner phase. When the content of the hydrogel-forming material is less than the above range, the hydrogel may not be formed. When the content exceeds the above range, the hydrogel may not be broken on the skin due to an increase in its hardness.

The salt includes at least one salt of an alkali metal salt and an alkaline earth metal salt, and a phosphate salt. The alkali metal salt and alkaline earth metal salt serve to cure the hydrogel, and the phosphate salt serves to delay the curing rate. Therefore, the hydrogel phase of the multilayer emulsion may stably be formed by mixing two kinds of the salts described above.

As the alkali metal salt and the alkaline earth metal salt, calcium salts, beryllium salts, magnesium salts, strontium salts, barium salts, radium salts, potassium salts, sodium salts, lithium salts, *etc.* may be used. For example, calcium chloride, calcium sulfate, calcium carbonate, potassium chloride, potassium bicarbonate, potassium hydroxide, sodium hydroxide, lithium hydroxide, *etc.* may be used, and these salts may be used alone or in combination of two or more.

One or more of the alkali metal salt and the alkaline earth metal salt may be included in an amount of 0.005 wt% to 0.5 wt%, for example, 0.01 wt% to 0.25 wt%, based on the total weight of the inner phase. When the content of one or more salts of the alkali metal salt and the alkaline earth metal salt is less than the above range, the degree of curing the hydrogel may be insufficient. When the content exceeds the above range, the reactivity may become too high, thus making it difficult to uniformly cure the hydrogel.

As the phosphate salt, tetrasodium pyrophosphate, trisodium phosphate, sodium tetraphosphate, sodium tripolyphosphate, sodium phosphate, disodium pyrophosphate, *etc.* may be used, and these may be used alone or in combination of two or more.

The phosphate salt may be included in an amount of 0.0025 wt% to 0.25 wt%, for example, 0.005 wt% to 0.125 wt% based on the total weight of the inner phase. When the content of the phosphate salt is less than the above range, the effect of delaying the curing rate may be insufficient, and thus the curing rate may be too fast. Therefore, the uniformity of particle size may be affected. When the content exceeds the above range, the effect of delaying the curing rate may be excessive and thus make the curing rate too slow, thereby preventing gel formation and making the formation of spherical particles difficult.

The inner phase may include active ingredients. For example, niacinamide, adenosine, hyaluronic acid, azelaic acid, ascorbic acid, kojic acid, glycolic acid, arbutin, peptides, *etc.* may be included.

Purified water may be included in an amount that satisfies the total weight (100 wt%) of the inner phase.

### Intermediate Phase

The intermediate phase of the multilayer emulsion cosmetic composition according to the present invention is an organogel layer, and it includes oil and an organogel-forming material.

The organogel-forming material serves to form an organogel structure, which is a three-dimensional network structure formed of a lipophilic solvent. When the organogel is formed in the intermediate phase, the network structure is maintained together with the oil and a thickener, and as a result, a stable multilayer emulsion may be formed without destruction of the interfacial film or a phase change even without using an emulsifier (surfactant).

As the organogel-forming material, amino acid derivatives such as *N*-lauroyl-L-glutamic acid, and α,γ-di-n-butylamine; dextrin fatty acid esters such as dextrin palmitate, dextrin stearate, dextrin palmitate/ethylhexanoate, and dextrin myristate; sucrose fatty acid esters such as sucrose palmitate, sucrose and stearate; fructooligosaccharide fatty acid esters such as inulin stearate and fructooligosaccharide 2-ethylhexanoate; monobenzyl sorbitol benzylidene derivatives such as monobenzylidene sorbitol, and dibenzylidene sorbitol; polyurethane-79; polyethylene; glyceryl behenate; glyceryl behenate/eicosadioate); stearoyl inulin; trihydroxystearin; polybutene; polyamide-2; polyamide-3; polyamide-5; polyamide-8; hydrogenated vegetable oil; isophorone diisocyanate copolymers (IPDI copolymers), *etc.* may be used, and these may be used alone or in combination by mixing two or more.

The organogel-forming material may be included in an amount of 5 wt% to 40 wt%, for example, 10 wt% to 20 wt% based on the total weight of the intermediate phase. When the content of the organogel-forming material is less than the above range, organogel may not be formed, or even if it is formed, it may be formed weakly, thus resulting in reduced particle maintaining ability and degree of stability. When the content exceeds the above range, the viscosity and hardness increase, thus not only impairing the feeling of use, but also making it difficult to prepare a multilayer emulsion.

The intermediate phase may include a stabilizer. The stabilizer exhibits a thickening effect and an effect of preventing oil sweating thereby enhancing the stabilization of the organogel and maintaining a stable multilayer emulsion.

As the stabilizer, disteardimonium hectorite, stearalkonium hectorite, quatemium-18 hectorite, dihydrogenated tallow benzylmonium hectorite, montmorillonite, bentonite, attapulgite, sepiolite, calcium silicate, silica, silica silylate, silica dimethyl silylate, polymethylsilsesquioxane/silica crosspolymers, mica, polymethyl methacrylate, nylon-6, iron oxide, tin oxide, methyl methacrylate crosspolymer, titanium dioxide, synthetic fluorphlogopite, polymethylsilsesquioxane, *etc.* may be used, and these may be used alone or in combination by mixing two or more.

The stabilizer may be included in an amount of 5 wt% to 40 wt%, for example, 10 wt% to 20 wt% based on the total weight of the intermediate phase. When the content of the stabilizer is less than the above range, the effect of enhancing the high temperature and long-term stability of the formed organogel may be insignificant. When the exceeds the above range, the viscosity and hardness increase, thus not only impairing the feeling of use, but also making it difficult to prepare a multilayer emulsion.

As the oil, any oil used in the art may be used without particular limitation. For example, as the oil, mineral oil, isohexadecane, isostearyl isostearate, isopropyl myristate, caprylic/capric triglyceride, diisopropyl adipate, isononyl isononanoate, isodecyl neopentanoate, cetyl ethyl hexanoate, octyldodecyl myristate, di-C12-13 alkyl maleate, pentaerythrityl tetraisostearate, pentaerythrityl tetraethyl hexanoate, diisostearyl maleate, isopropyl palmitate, C12-15 alkyl benzoate, dicaprylic carbonate, dicaprylic ether, 2-octyldodecanol, dimethicone, lauryl dimethicone, cyclopentasiloxane, cyclohexasiloxane, trisiloxane, methyl trimethicone, amo dimethicone, phenyl trimethicone, phenyl methicone, dimethicone copolyol, dimethicone copolyol acetate, silicone glycol copolyol, dimethiconol, methyl polysiloxane, methylphenyl polysiloxane, dimethicone copolyol methyl ether, methylcyclo polysiloxane, dimethyl polysiloxane, hexamethyl disiloxane, aminopropyl dimethicone, caprylyl methyl polysiloxane, simethicone, *etc.* may be used, and these may be used alone or in combination by mixing two or more.

The intermediate phase may include active ingredients. For example, it may include retinyl palmitate, retinol, retinal, tretinoin, retinoic acid, adapalene, tazarotene, isotretinoin, calciferol, tocopherol, phytonadione, *etc.*

The oil may be included in an amount that satisfies the total weight of the intermediate phase (100 wt%).

The organogel of the intermediate phase may have a hardness of 5 dyne/cm² to 60 dyne/cm², for example, 10 dyne/cm² to 30 dyne/cm². When the hardness of the organogel is less than the above range, gelation does not proceed and it thus becomes difficult to maintain the particle shape, and as a result, the multilayer emulsion structure may not be maintained. In contrast, when the hardness exceeds the above range, the hardening rate becomes fast, and thus it is not suitable for forming a multilayer emulsion, and spreadability on the skin may be poor.

### Outer Phase

The outer layer of the multilayer emulsion cosmetic composition according to the present invention is a water layer and includes purified water.

The outer phase may include a water-soluble thickener. The water-soluble thickener present in the outer phase plays a role of maintaining particle stability (sinking/floating) in the intermediate phase and the inner phase.

As the water-soluble thickener, a carbomer, sodium polyacryloyldimethyl taurate, polyacrylate crosspolymer-11, an acrylate copolymer, an acrylate/C 10-30 alkyl acrylate crosspolymer, a carbomer ammonium acryloyldimethyltaurate/vinylpyrrolidone (VP) copolymer, sodium polyacrylate, cellulose gum, hydroxyethylcellulose, ethylcellulose, carboxymethyl hydroxypropyl guar, xanthan gum, gum arabic, guar gum, *etc.* may be used, and these may be used alone or in combination by mixing two or more.

The water-soluble thickener may be included in an amount of 0.05 wt% to 10 wt%, for example, 0.1 wt% to 5wt% based on the total weight of the outer phase. When the content of the water-soluble thickener is less than the above range, the viscosity may be low and thus the particle stability may be poor. When the content exceeds the above range, the viscosity may be high, and thus the feeling of use such as spreadability and stickiness may be deteriorated, and it may be difficult to prepare a multilayer emulsion.

The outer layer may include polyol. As the polyol, glycerin, 1,3-butylene glycol, propylene glycol, polyethylene glycol, sorbitol, pentylene glycol, methyl propanediol, *etc.* may be used, and these may be used alone or in combination by mixing two or more.

The polyol may be included in an amount of 0.5 wt% to 40 wt%, for example, 1 wt% to 20 wt% based on the total weight of the outer phase.

As the outer phase, a chelating agent, a pH adjusting agent, a solvent, a film forming agent, a moisturizer, an antiseptic stabilizer, a skin conditioning agent, a functional cosmetic active ingredient, *etc.* may be used. As the preservative, 1,2-hexanediol, ethylhexyl glycerin, hydroxyacetophenone, glyceryl caprylate, caprylyl glycol, phenoxyethanol, *etc.* may be used. As the chelating agent, disodium EDTA, trisodium EDTA, tetrasodium EDTA, *etc.* may be used. As the pH adjusting agent, tromethamine, triethanolamine, imidazolidinyl urea, *etc.* may be used.

The multilayer emulsion cosmetic composition of the present invention may be prepared in any one formulation of a lotion, an essence, a cream, a serum, a mask solution, an ampoule, and a pack, but is not limited thereto.

The multilayer emulsion cosmetic composition of the present invention may be prepared according to various methods known in the art. For example, it may be prepared by the following method.

A hydrogel phase containing purified water, a hydrogel-forming material, and a salt; and an organogel phase containing oil, an organogel-forming material, and a stabilizer, as necessary, are prepared, respectively. The prepared hydrogel phase is added to the organogel phase and stirred to prepare a W(hydrogel)/O(organogel) emulsion formulation. A W/O/W multi-emulsion formulation may be prepared by injecting the prepared W/O emulsion formulation into an outer phase followed by stirring.

### Embodiments for Implementing the Invention

Hereinafter, the present invention will be described in more detail through examples. However, the following examples are only for facilitating the understanding of the present invention, and the scope of the present invention is not limited to these examples in any sense.

### [Examples 1 to 3: Preparation of intermediate phase]

After preparing the intermediate phase of each example according to the compositions shown in Table 1 below, the properties thereof were measured by the following method.

### [Comparative Examples 1 to 3: Preparation of intermediate phase]

Except for the compositions shown in Table 1 below, the intermediate phase was prepared in the same manner as in Examples 1 to 3, and properties thereof were measured.

### Preparation of O/W formulation

An outer phase, which is formed of 0.2 wt% of carbomer, 0.2 wt% of tromethamine, 10 wt% of polyol, 1.5 wt% of 1,2-hexanediol, 0.05 wt% of ethylhexyl glycerin, and the remaining balance of water (up to 100 wt%) was prepared, and then, intermediate phases of each Example and Comparative Example were added to the outer phase to prepare O/W formulations. In order to confirm the stability of the intermediate phase in the presence of outer phase while excluding the involvement of the inner phase, the properties of the intermediate phase in the O/W formulations were evaluated.

### Presence/absence of organogel formation

The presence/absence of formation of an organogel in the intermediate phase in the O/W formulations of each Example and Comparative Example was observed with the naked eye or using a microscope.

### Shape of organogel particles

The shape of the organogel particles in the O/W formulations of each Example and Comparative Example was observed using a microscope.

### [Evaluation standard]

∘: a round shape with a stable particle interface
X: a distorted shape with an unstable particle interface

### Hardness

Using a FUDOH rheometer, the hardness of the intermediate phases of each Example and Comparative Example was measured (under 1 mmΦ, 30 cm/mim, 10 mm depth conditions).

### High temperature stability

The O/W formulations prepared in Examples and Comparative Examples were left at a temperature of 40°C to 60°C for one month, and it was checked whether oil exudes using a microscope to evaluate high-temperature stability.

### [Evaluation standard]

●: There was no oil exudation over the entire temperature range.
∘: There was slight oil exudation at 60°C.
X: There was oil exudation at a temperature below 60°C.

**[Table 1]**

| Component (wt%) | Ex. 1 | Ex. 2 | Ex. 3 | Comp.Ex. 1 | Comp.Ex. 2 | Comp.Ex. 3 |
|---|---|---|---|---|---|---|
| Oil (Octyldodecanol) | to 100 | to 100 | to 100 | to 100 | to 100 | to 100 |
| Organogel-Forming Material (Polyamide-8) | 10 | 20 | 10 | 2.5 | 50 | |
| Stabilizer (Hectorite) | 10 | | | | | 10 |
| Presence/Absence of Organogel Formation | O | O | O | O | O | X |
| Shape of Organogel Particles | O | O | O | X | X | |
| Hardness | 22 | 20 | 10 | 2 | 65 | 1 |
| High Temperature Stability | ● | ○ | ○ | X | X | |

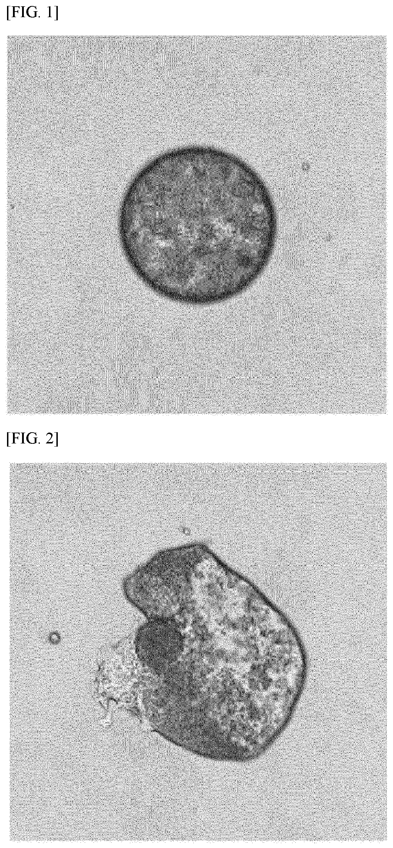

As shown in Table 1, the intermediate phases of Examples 1 to 3 according to the present invention formed an organogel having a stable round shape of a particle interface, and exhibited excellent hardness and high temperature stability. In particular, the intermediate phase of Example 1, which further includes a stabilizer, formed an organogel having a stable round shape of a particle interface (FIG. 1). The high temperature stability was shown to be very excellent.

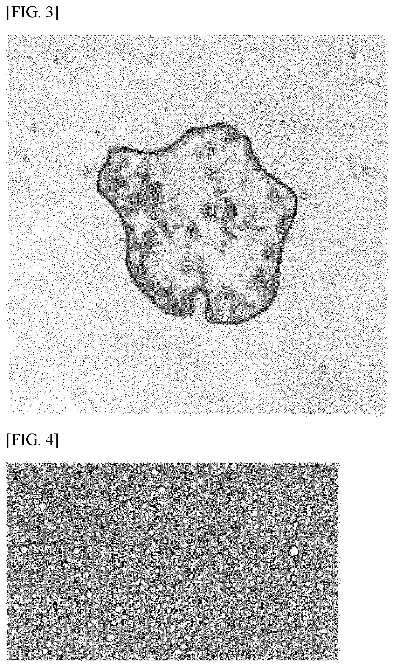

In contrast, in the case of Comparative Example 1 including the organogel-forming material in an amount less than the range of the present invention, the hardness of the intermediate phase was shown to be very low, and as a result, the particles were not maintained at high temperatures and were crushed and eventually burst, that is, a phenomenon in which the emulsion is broken occurred (FIG. 2). The intermediate phase of Comparative Example 2 including the organogel-forming material in an amount exceeding the range of the present invention formed an organogel, but the interface of the particles was unstable and was formed in a distorted form. When the hardness is too high as in Comparative Example 2, it becomes sensitive to temperature and hardens quickly, and thus the particles harden during the preparation of the multilayer emulsion, thereby making it difficult to form the particle in a round shape, and since it is hard and does not break on the skin, it becomes difficulty to apply the same and the phenomenon of oil exudation at high temperature increases. In contrast, the intermediate phase of Comparative Example 3 including only a stabilizer without including an organogel-forming material did not form an organogel (FIG. 3).

### [Examples 4 to 6: Preparation of W/O formulations]

After preparing the inner phase of each Example according to the compositions shown in Table 2 below, the inner phases (hydrogel) of Examples 4 to 6 were each added to the intermediate phase (organogel) prepared in Example 1 and the mixture was stirred with a homomixer to prepare W(hydrogel)/O(organogel) emulsion formulations. The physical properties of the prepared W/O formulations of each Example were measured by the method described below.

### [Comparative Examples 4 and 5: Preparation of W/O Formulations]

Except for the compositions described in Table 2 below, W/O formulations were prepared in the same manner as in Examples 4 to 6, and properties thereof were measured.

### Presence/Absence of hydrogel formation

The presence/absence of hydrogel formation in the inner phase within the W/O formulations of each Example and Comparative Example was observed with the naked eye or using a microscope.

### Particle shape/particle uniformity of hydrogel

The shape of the particles of the hydrogels of each Example and Comparative Example and presence/absence of uniform distribution of the particles in the intermediate phase were observed using an optical microscope.

**[Table 2]**

| Components | Ex. 4 | Ex. 5 | Ex. 6 | Comp. Ex. 4 | Comp. Ex. 5 |
|---|---|---|---|---|---|
| Water | to 100 | to 100 | to 100 | to 100 | to 100 |
| Hydrogel-Forming Material (Gellan Gum) | 1 | 1 | 1 | 1 | 1 |
| Salt 1 (Calcium Chloride) | 0.1 | 0.1 | 0.1 | 0.2 | |
| Salt 2 (Tetrasodium Pyrophosphate) | 0.1 | 0.001 | 1 | | 0.2 |
| Presence/Absence of Hydrogel Formation | O | O | O | O | X |
| Particle Shape of Hydrogel | spherical type | spherical | rod type | spherical | |
| Uniformity of Hydrogel Particle Size | uniform | non-uniform | uniform | non-uniform | |

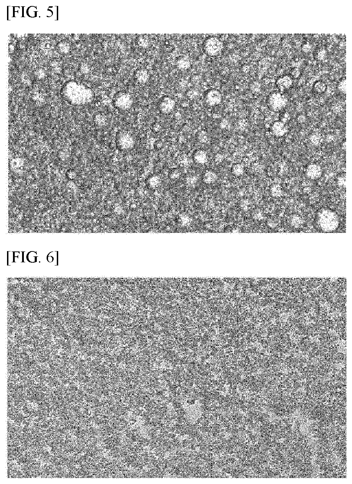

As shown in Table 2, the inner phase within the W/O formulations of Examples 4 to 6 according to the present invention formed a hydrogel (FIGS. 4 to 6). In particular, the inner phase within the W/O formulation of Example 4 containing 0.1 wt% of a phosphate salt formed a spherical hydrogel with a uniform particle size (FIG. 4).

In contrast, the inner phase in the W/O formulation of Comparative Example 4 containing only a calcium salt of the two salts according to the present invention formed a hydrogel, but was immediately gelled and thus the particles were not uniformly formed. Meanwhile, the inner phase in the W/O formulation of Comparative Example 5 containing only the phosphate salt of the two salts according to the present invention did not form a hydrogel. Therefore, the W/O formulations of Comparative Examples 4 and 5 were not used for preparing multilayer emulsions.

### [Examples 7 to 9: Preparation of multilayer emulsion (W/O/W)]

An outer phase, which is formed of 0.2 wt% of carbomer, 0.2 wt% of tromethamine, 10 wt% of polyol, 1.5 wt% of 1,2-hexanediol, 0.05 wt% of ethylhexyl glycerin, and the remaining balance of water (up to 100 wt%) was prepared. The W/O emulsion formulations of Examples 4 to 6 were each added to the prepared outer phase and stirred with an Aji mixer to prepare W/O/W multilayer emulsions of Examples 7 to 9.

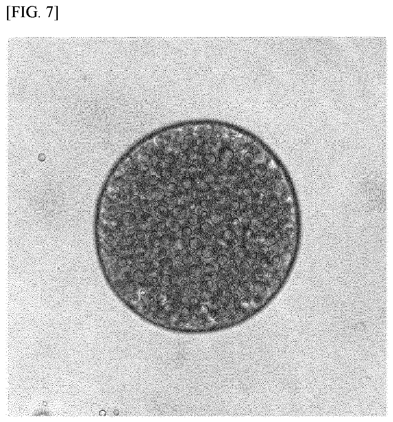

As a result of observing the W/O/W multilayer emulsions of each Example using an optical microscope, it was confirmed that in all the emulsions of Examples 7 to 9, the particles of the intermediate phase were uniformly distributed in the outer phase while including the inner phase (FIG. 7), thereby confirming the formation of stable multilayer emulsions.

### Industrial Applicability

The present invention provides a multilayer emulsion cosmetic composition that exhibits excellent stability due to high stability of the interfacial film between a water phase and an oil phase without containing a surfactant. Since the cosmetic composition according to the present invention does not contain a surfactant, it is less irritating to the skin and is environmentally-friendly, and as a multilayer emulsion formulation, it has excellent applicability, spreadability, and feeling of use, as well as excellent makeup durability and water resistance. In addition, the cosmetic composition of the present invention is suitable for simultaneously stabilizing oil-soluble and water-soluble active ingredients in one formulation because the intermediate phase and the inner phase are not brought into contact with air due to the structural characteristics of the multilayer emulsion.

## Claims

1. A multilayer emulsion cosmetic composition comprising an inner phase of a hydrogel layer, an intermediate phase of an organogel layer, and an outer phase of a water layer,
wherein the inner phase comprises purified water, a hydrogel-forming material, and a salt,
the salt comprising one or more salts among an alkali metal salt and an alkaline earth metal salt, and a phosphate salt,
the intermediate phase comprises an oil and an organogel-forming material, and
the organogel-forming material is contained in an amount of 5 wt% to 40 wt% based on the total weight of the intermediate phase.

2. The multilayer emulsion cosmetic composition of claim 1, wherein the hydrogel-forming material is one or more selected from the group consisting of gellan gum, agar, carrageenan, alginate, pectin, gelatin, xanthan gum, locust bean gum (LBG), guar gum, starch, chitosan, cellulose, and collagen.

3. The multilayer emulsion cosmetic composition of claim 1, wherein the alkali metal salt and the alkaline earth metal salt are one or more selected from the group consisting of a calcium salt, a beryllium salt, a magnesium salt, a strontium salt, a barium salt, a radium salt, a potassium salt, a sodium salt, and a lithium salt.

4. The multilayer emulsion cosmetic composition of claim 1, wherein the organogel-forming material is one or more selected from the group consisting of *N*-lauroyl-L-glutamic acid, α,γ-di-*n*-butylamine, dextrin palmitate, dextrin stearate, dextrin palmitate/ethylhexanoate, dextrin myristate, sucrose palmitate, sucrose stearate, inulin stearate, fructooligosaccharide 2-ethylhexanoate, monobenzylidene sorbitol, dibenzylidene sorbitol, polyurethane-79, polyethylene, glyceryl behenate, glyceryl behenate/eicosadioate, stearoyl inulin, trihydroxystearin, polybutene, polyamide-2, polyamide-3, polyamide-5, polyamide-8, hydrogenated vegetable oil, and an isophorone diisocyanate (IPDI) copolymer.

5. The multilayer emulsion cosmetic composition of claim 1, wherein the multilayer emulsion cosmetic composition comprises 0.05 wt% to 10 wt% of the hydrogel-forming material, 0.005 wt% to 0.5 wt% of one or more salts among the alkali metal salt and the alkaline earth metal salt,and 0.0025 wt% to 0.25 wt% of the phosphate salt based on the total weight of the inner phase.

6. The multilayer emulsion cosmetic composition of claim 1, wherein the multilayer emulsion cosmetic composition further comprises 5 wt% to 40 wt% of a stabilizer based on the total weight of the intermediate phase.

7. The multilayer emulsion cosmetic composition of claim 1, wherein the multilayer emulsion cosmetic composition further comprises 0.05 wt% to 10 wt% of a water soluble thickener based on the total weight of the outer phase.

8. The multilayer emulsion cosmetic composition of claim 1, wherein the hardness of the organogel of the intermediate phase is 5 dyne/cm² to 60 dyne/cm².
